# EUROPEAN PATENT APPLICATION

(11) **EP 3 333 167 A1**
(43) Date of publication of application: **13.06.2018**
(21) Application number: 16203212.2
(22) Date of filing: 09.12.2016
(51) Int. Cl.: C07D 471/04, A61K 31/437, A61K 31/496, A61P 35/00, A61K 9/20

(54) **SOLID FORMS OF VENETOCLAX**

(71) Applicant: LEK Pharmaceuticals d.d., 1526 Ljubljana (SI)
(72) Inventor: The designation of the inventor has not yet been filed
(74) Representative: Kluschanzoff, Harald

(57) **Abstract**

The present invention relates to novel forms of 4-(4-{[2-(4-chlorophenyl)-4,4-dimethylcyclohex-1 -en- 1 -yl]methyl}piperazin-1 -yl)-N-({3-nitro-4-[(tetrahydro-2H-pyran-4-ylmethyl)amino]phenyl}sulfonyl)-2-(1H-pyrrolo[2,3-b]pyridin-5-yloxy)benzamide (also known as venetoclax) as well as to the preparation thereof. One novel polymorph is an anhydrous crystalline form of venetoclax (referred to herein as "Form S"). Another novel polymorph is a crystalline hydrate of venetoclax (referred to herein as "Form HyD"). The present invention also discloses a novel amorphous form of venetoclax having a low solvent content, and refers to a pharmaceutical composition comprising said novel forms of venetoclax.

Moreover, the present invention relates to the use of Form HyD as an intermediate for the preparation of crystalline Form S.

Finally, the present invention refers to the novel polymorphs, or the pharmaceutical composition comprising the novel polymorphs of venetoclax or amorphous form of venetoclax, for use in a method of preventing or treating a disease that is characterized by apoptotic dysfunction and/or overexpression of an anti-apoptotic Bcl-2 family protein.

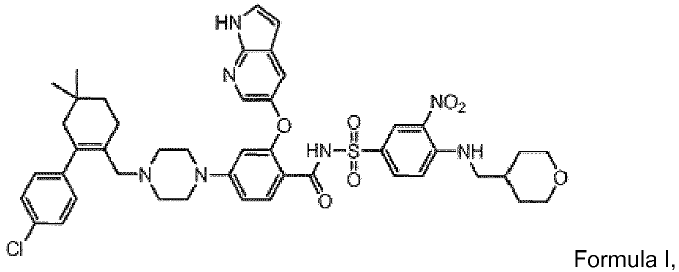

## Description

### Field of the invention

The present invention relates to novel forms of 4-(4-{[2-(4-chlorophenyl)-4,4-dimethylcyclohex-1 -en- 1 -yl]methyl}piperazin-1 -yl)-N-({3-nitro-4-[(tetrahydro-2H-pyran-4-ylmethyl)amino]phenyl}sulfonyl)-2-(1H-pyrrolo[2,3-b]pyridin-5-yloxy)benzamide (also known as venetoclax) as well as to the preparation thereof. One novel polymorph is an anhydrous crystalline form of venetoclax (referred to herein as "Form S"). Another novel polymorph is a crystalline hydrate of venetoclax (referred to herein as "Form Hyd"). One further novel form is an amorphous form of venetoclax having a low solvent content.

The present invention also refers to a pharmaceutical composition comprising said forms of venetoclax.

Moreover, the present invention relates to the use of Form Hyd as an intermediate for the preparation of Form S.

Finally, the present invention refers to the novel polymorphs, or the pharmaceutical composition comprising the novel polymorphs of venetoclax or amorphous form of venetoclax, for use in a method of preventing or treating a disease that is characterized by apoptotic dysfunction and/or overexpression of an anti-apoptotic Bcl-2 family protein.

### Background of the invention

4-(4-{[2-(4-chlorophenyl)-4,4-dimethylcyclohex- 1 -en- 1 -yl]methyl}piperazin-I-yl)-N-({3-nitro-4-[(tetrahydro-2Hpyran-4-ylmethyl)amino]phenyl}sulfonyl)-2-(IH-pyrrolo[2,3-b]pyridin-5-yloxy)benzamide, also known as venetoclax, is a BH3-mimetic and acts as Bcl-2 (B-cell lymphoma-2) inhibitor. It blocks the anti-apoptotic Bcl-2 protein, therefore leading to programmed cell death (apoptosis) of affected cells. Currently, venetoclax is marketed under the trade name Venclexta.

Venetoclax is represented by the following general Formula I:

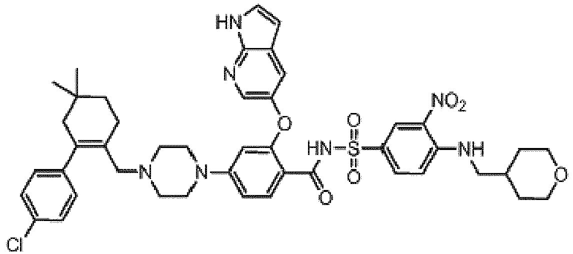

WO 2012/071336 discloses various salts and crystalline forms of venetoclax per se as well as pharmaceutical compositions comprising the same. Examples of crystalline forms of venetoclax that are disclosed in WO 2012/071336 are Form A and Form F.
Polymorphism is a phenomenon relating to the occurrence of different crystalline forms for one molecule. There may be several different crystalline forms for the same molecule with distinct crystal structures and varying in physical properties like melting point, XRPD pattern and FTIR spectrum. These polymorphs are thus distinct solid forms which share the molecular formula of the compound from which the crystals are made up, however they may have distinct advantageous physical properties such as e.g. chemical stability, physical stability, hygroscopicity, solubility, dissolution rate, morphology or bioavailability. In addition, the preparation process of a crystalline form plays an important role in the development of an active pharmaceutical ingredient. It is essential that the crystallization process is robust and reliably produces the desired crystalline form in polymorphically pure form.

The acceptable amount of solvents in an active pharmaceutical ingredient (API) is strictly regulated e.g. by the ICH guideline for residual solvents. Hence, it is a desire to provide APIs that comprise an amount of solvent as low as possible.

In addition, an active pharmaceutical ingredient is preferably non-hygroscopic in order to ensure the chemical and physical quality, e.g., against interconversion to another solid form, during the storage of the active substance itself and during the shelf-life of a solid finished dosage form containing the active substance without the need of special and expensive packaging.

Furthermore, the bioavailability of a compound intended to be administered orally, is dependent on the compound's solubility as well as the compound's permeability according to the biopharmaceutical classification system (BCS). Therefore, a drug substance having high solubility which is consequently highly bioavailable is desired.

It is thus an object of the present invention to provide improved forms/dosage forms of venetoclax, preferably polymorphs, that are improved with regard to the above described properties.

### Summary of the invention

The present invention provides the following aspects, subject-matters and preferred embodiments, which respectively taken alone or in combination, contribute to solving the object of the present invention:
The present invention refers to new crystalline Form S of venetoclax, wherein venetoclax is represented by Formula I (4-(4-{[2-(4-chlorophenyl)-4,4-dimethylcyclohex-1 1 -en- 1-yl]methyl}piperazin-1-yl)-N-({3-nitro-4-[(tetrahydro-2H-pyran-4-ylmethyl)amino]phenyl}sulfonyl)-2-(1 H-pyrrolo[2,3-b]pyridin-5-yloxy)benzamide) 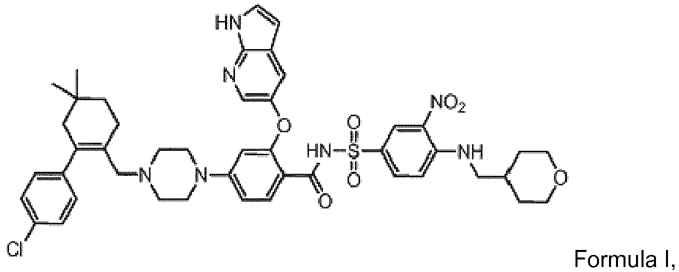 characterized by an X-ray powder diffraction pattern with peaks at 2-theta angles of 6.3±0.2 degrees 2theta, 11.4±0.2 degrees 2theta, 13.3±0.2 degrees 2theta, 14.3±0.2 degrees 2theta, 16.9±0.2 degrees 2theta, and 19.2±0.2 degrees 2theta, when using Cu-Kα radiation.

Form S of venetoclax exhibits improved properties when compared to prior art crystalline forms of venetoclax, e.g. in terms of stability, solvent content, hygroscopicity, and/or processability.

The present invention further provides a method for the preparation of the crystalline Form S of the present invention, comprising the step:
(B) drying Form HyD of venetoclax, characterized by an X-ray powder diffraction pattern with peaks at 2-theta angles of 5.2±0.2 degrees 2theta, 9.2±0.2 degrees 2theta, 15.0±0.2 degrees 2theta, 21.3±0.2 degrees 2theta, 21.8±0.2 degrees 2theta, when using Cu-Kα radiation, at a temperature and for a time period suitable to obtain crystalline Form S.

Form HyD is comparably easy to desolvate and this is a superior property. Form HyD thus exhibits advantageous and beneficial properties such as having a low water/solvent content.

It is known to a person skilled in the art that venetoclax is very prone to solvate formation. There is thus no reason for a skilled person to anticipate that an MTBE solvate would be unstable and that it would decompose into an intermediate hydrated form which would be easy to desolvate. Already for this reason, the selection of the intermediate Form HyD for the production of Form S is not obvious.

The present invention further refers to the use of crystalline Form S of the present invention, for the preparation of a pharmaceutical composition, preferably comprising an amorphous form of venetoclax.
Crystalline Form S exhibits a low solvent content. This low solvent content of Form S is due to the low solvent content of Form HyD on the one hand, and on the other hand, this low solvent content is further passed on the pharmaceutical composition that is prepared by using crystalline Form S.

Thus, the present invention also provides new crystalline Form HyD of venetoclax, represented by Formula I (4-(4-{[2-(4-chlorophenyl)-4,4-dimethylcyclohex- 1 -en- 1 -yl]methyl}piperazin-I-yl)-N-({3-nitro-4-[(tetrahydro-2Hpyran-4-ylmethyl)amino]phenyl}sulfonyl)-2-(IH-pyrrolo[2,3-b]pyridin-5-yloxy)benzamide) 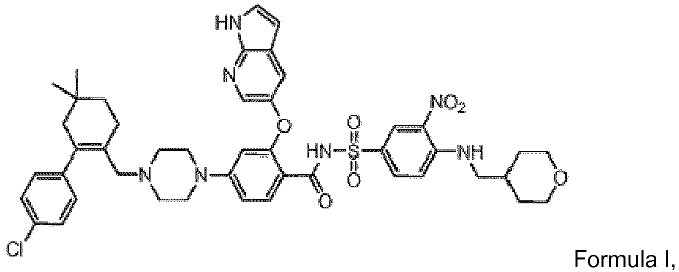 characterized by an X-ray powder diffraction pattern with peaks at 2-theta angles of 5.2±0.2 degrees 2theta, 9.2±0.2 degrees 2theta, 15.0±0.2 degrees 2theta, 21.3±0.2 degrees 2theta, 21.8±0.2 degrees 2theta, when using Cu-Kα radiation.

Additionally, the present invention refers to the use of the crystalline Form HyD as defined in the present invention as an intermediate for the preparation of crystalline Form S of the present invention.

The present invention further refers to amorphous venetoclax being essentially free of organic solvents, preferably free of any solvents (including inorganic solvents and water), and to amorphous venetoclax obtainable or obtained by conversion of crystalline Form S of venetoclax as disclosed herein, wherein the conversion is performed in the absence of solvents; conversion can be performed simply by melting the crystalline Form S.

The present invention thus also refers to pharmaceutical composition comprising an amorphous form of venetoclax as disclosed herein, an amorphous solid dispersion of venetoclax as disclosed herein, the crystalline Form HyD as disclosed herein, or the crystalline Form S as disclosed herein, and one or more pharmaceutically acceptable excipients.

Finally, the present invention relates to Form S, Form HyD, amorphous form of venetoclax (respectively as disclosed herein), and to the pharmaceutical composition of the present invention, for use in a method of preventing or treating a disease that is characterized by apoptotic dysfunction and/or overexpression of an anti-apoptotic Bcl-2 family protein.

### Definitions

As used herein the term "room temperature" indicates that the applied temperature is not critical and that no exact temperature value has to be kept. Usually, "room temperature" is understood to mean temperatures of about 15 °C to about 25 °C (see e.g. EU Pharmacopoeia 7.5, 1.2 (2012)).

As used herein the term "ambient temperature" is understood to mean temperatures of about 20 °C to about 25°C.

The term "solvate" as used herein describes a crystalline compound in which solvent molecules are incorporated into the crystal lattice of the compound in a stoichiometric or non-stoichiometric manner. If the solvent molecules are water the term "hydrate" is used herein.

The term "non-hygroscopic" as used herein indicates that the increase in mass of a drug substance between about 0% to 80% relative humidity is less than 0.2%.

The term "essentially", "essential", "approximately" and "about" in the context of the present invention denote an interval of accuracy that the person skilled in the art will understand to still ensure the technical effect of the feature in question. The term typically indicates deviation from the indicated numerical value of ±10%, and preferably ±5%.

The expression "forms of venetoclax of the present invention" defines the different forms of venetoclax of the present invention, i.e. crystalline Form S and Form HyD, and the amorphous form of venetoclax. In a pharmaceutical composition or dosage form of the present invention, such as a tablet, venetoclax can be present in any of these forms. Preferably, venetoclax is present in a pharmaceutical composition or dosage from in one form as disclosed in the present invention.

The tolerance margin of specified or selected XRPD peaks is considered to be, and shall mean herein, 0.2 degrees 2 theta.

### Brief description of the drawings

- Figure 1:: XRPD pattern of Form S of venetoclax.

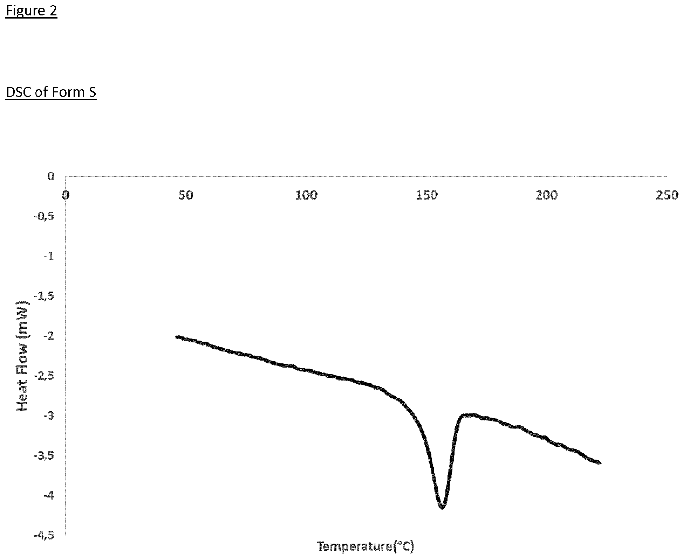

- Figure 2:: DSC measurement (Method 1) of Form S of venetoclax, wherein a single endothermic event is shown at 156°C.

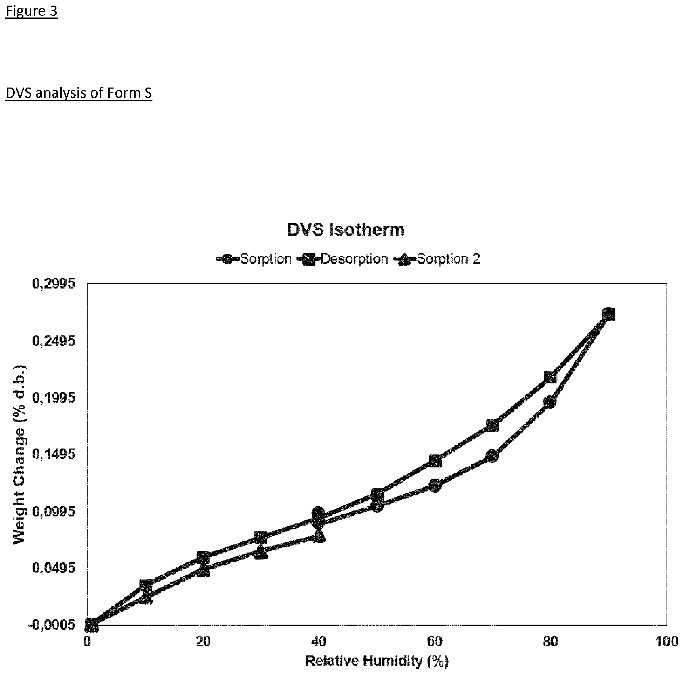

- Figure 3:: DVS ("Dynamic Vapour Sorption") analysis of Form S of venetoclax, showing a weight gain of 0.3% between 0 and 90% relative humidity. DVS measures the weight increase of a sample at different relative humidities.

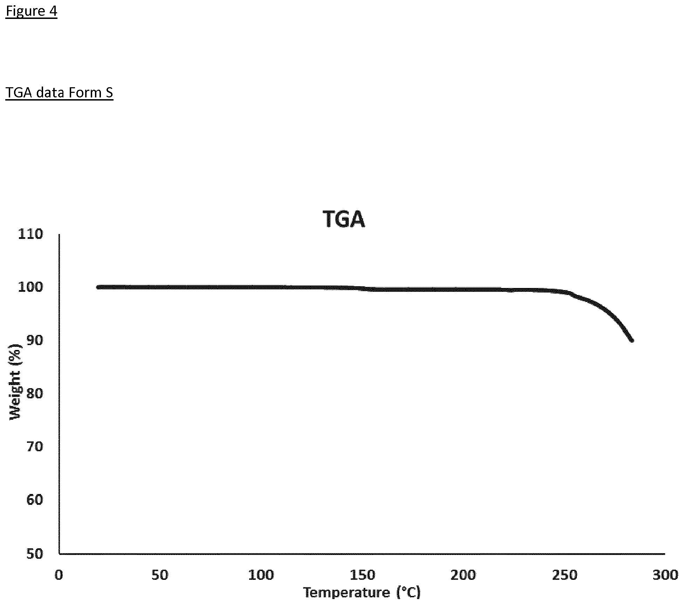

- Figure 4:: TGA analysis ("Thermogravimetric Analysis") of Form S of venetoclax.
- Figure 5:: XRPD pattern of Form HyD.
- Figure 6:: Full peak list of Form HyD.
- Figure 7:: DSC measurement (Method 2) of Form A of venetoclax, wherein a single endothermic event is shown at 140°C. The X-axis shows the temperature / °C and the Y-axis shows peak heights / Wg⁻¹. Endothermic events are plotted up.
- Figure 8:: DSC measurement (Method 2) of Form S of venetoclax, wherein a single endothermic event is shown at 163°C. The X-axis shows the temperature / °C and the Y-axis shows peak heights / Wg⁻¹. Endothermic events are plotted up.

### Detailed description of the invention

It has unexpectedly been found that it is possible to provide new forms of venetoclax which exhibit desirable properties, in particular regarding their low solvent content. Further benefits of the crystalline forms of the present invention are, for instance, improved stability and processability, as well as the possibility to be obtainable in high yields and purity.

One new crystalline form of venetoclax (referred to herein as "Form S") exhibits a comparably low content of residual solvents. This low content of residual solvent is particularly advantageous as, for instance, it passes to the low residual solvent content of an amorphous venetoclax preparation, when Form S is used e.g. as the starting material for preparing a finished dosage form (FDF) comprising an amorphous dispersion comprising venetoclax.

The crystalline Form S of the present invention may particularly show unexpected advantages and improvements when compared e.g. to the known Form A of venetoclax of WO 2012/071336 A1. For instance, it is assumed that Form S exhibits a reduced content (amount) of solvent, i.e. water and organic solvent, when compared to known Form A. It is also assumed that it is less hygroscopic. This contributes for instance to improved stability and/or processability. Thus, Form S of the present invention is especially suitable for the preparation of a pharmaceutical composition such as a solid medicament, or as an intermediate for preparing another low solvent form of venetoclax. Since Form S has a reduced solvent content it is particularly suitable as a starting material for the preparation of a dosage form comprising an amorphous form of venetoclax. This is particularly useful as it is very difficult, if possible at all, to remove residual solvent content of the directly obtained amorphous venetoclax, in order to arrive at a level of residual solvent content that is comparable to the level that is obtained when applying the indirect method according to the present invention.
Additionally, a low solvent and/or water content is advantageous when subjecting the respective forms to further processing methods such as melt extrusion. Moreover, the use of essentially anhydrous (preferably anhydrous), non-hygroscopic and low solvent content feed materials such as the forms of venetoclax of the present invention provides for an improved processability as for instance the need for equipment modification e.g. for the removal of volatiles (vent installation) and effects on API-polymer miscibility can be avoided.

Moreover it was found that the melting point of Form S and the heat of fusion are both higher than the corresponding values for Form A. From this it can deduced that A and S are monotropically related and that Form S is thermodynamically more stable than Form A (Polymorphism in the Pharmaceutical Industry, Rolf Hilfiker Ed., Wiley-VCH Verlag GmbH & Co. 2006, p 31ff). A thermodynamically stable API has advantages over other thermodynamically less stable forms as it will not convert to other crystalline forms during extended periods of storage.

It was unexpectedly found that slurrying venetoclax in t-butyl-methyl ether (MTBE) produces a MTBE solvate which has been found to be highly metastable. Upon filtration, the solvate desolvates and transforms into another new crystalline form of venetoclax (referred to herein as "Form HyD"). Therein, atmospheric moisture replaces part of the MTBE, thus reducing the organic solvent content.
It has been surprisingly found that Form HyD is comparably easy to desolvate.
Surprisingly, the water in Form HyD can then be removed to provide Form S having reduced organic solvent content and reduced water content. Accordingly, due to the crystalline structure of Form S, water and solvents are 'squeezed out'. The crystalline hydrate, Form HyD, is therefore a valuable intermediate for the preparation of the novel polymorph Form S of venetoclax.

Accordingly, Form S and Form HyD of the present application commonly share the property of having a low solvent content. This property is also shared by amorphous venetoclax of the present invention.

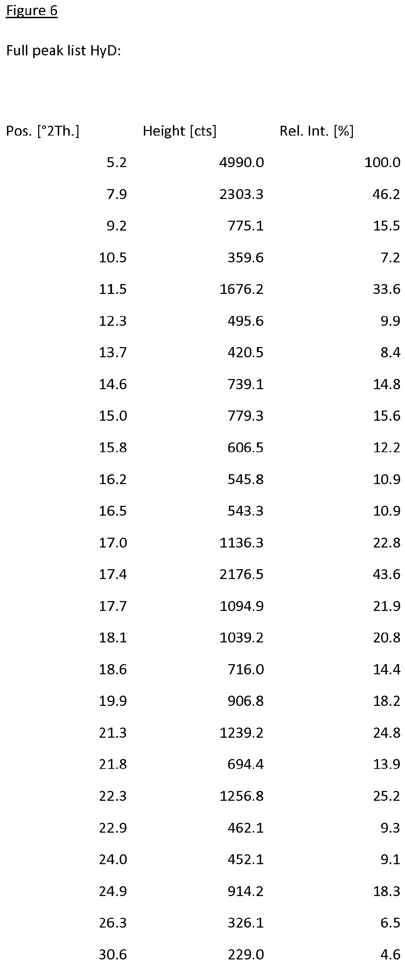

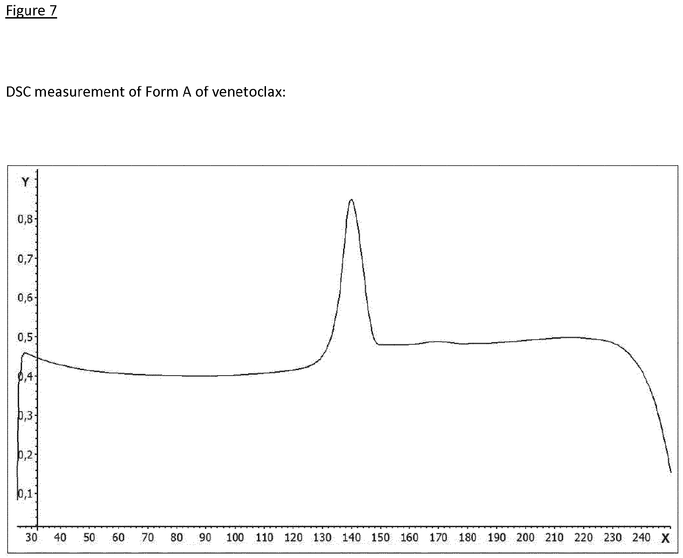

The present invention provides the following aspects, subject-matters and preferred embodiments, which respectively taken alone or in combination, contribute to solving the object of the present invention.
1. Crystalline Form S of venetoclax, wherein venetoclax is represented by Formula I (4-(4-{[2-(4-chlorophenyl)-4,4-dimethylcyclohex-1 -en- 1 -yl]methyl}piperazin-1 -yl)-N-({3-nitro-4-[(tetrahydro-2H-pyran-4-ylmethyl)amino]phenyl}sulfonyl)-2-(1H-pyrrolo[2,3-b]pyridin-5-yloxy)benzamide) 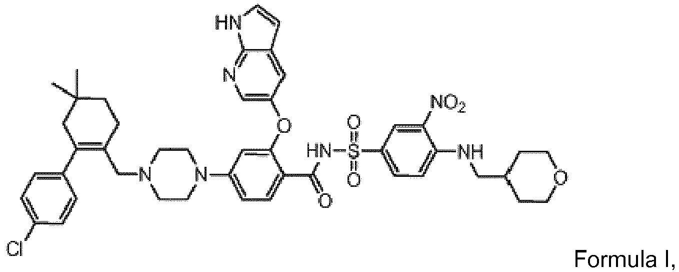 characterized by an X-ray powder diffraction pattern with peaks at 2-theta angles of 6.3±0.2 degrees 2theta, 11.4±0.2 degrees 2theta, 13.3±0.2 degrees 2theta, 14.3±0.2 degrees 2theta, 16.9±0.2 degrees 2theta, and 19.2±0.2 degrees 2theta, when using Cu-Kα radiation.
   Venetoclax is used herein in its free base form. The crystalline Form S of the present invention is an essentially anhydrous and essentially non-solvated crystalline form.
2. The crystalline Form S of item 1, wherein the X-ray powder diffraction pattern has peaks at 2-theta angles of 6.3±0.2 degrees 2theta, 7.1±0.2 degrees 2theta, 7.8±0.2 degrees 2theta, 8.6±0.2 degrees 2theta, 11.4±0.2 degrees 2theta, 11.7±0.2 degrees 2theta, 12.7±0.2 degrees 2theta, 13.3±0.2 degrees 2theta, 14.3±0.2 degrees 2theta, 14.7±0.2 degrees 2theta, 15.5±0.2 degrees 2theta, 16.4±0.2 degrees 2theta, 16.9±0.2 degrees 2theta, 17.9±0.2 degrees 2theta, 18.2±0.2 degrees 2theta, 19.2±0.2 degrees 2theta, 19.9±0.2 degrees 2theta, 22.0±0.2 degrees 2theta, 22.4±0.2 degrees 2theta, 22.9±0.2 degrees 2theta, 24.2±0.2 degrees 2theta, 26.8±0.2 degrees 2theta, 28.6±0.2 degrees 2theta, and 29.8±0.2 degrees 2theta, when using Cu-Kα radiation.
3. The crystalline Form S according to item 1 or 2, having a water content of less than 1 wt.-%, preferably less than 0.1 wt.-%, and more preferably, there is no detectable amount of water present.
4. The crystalline Form S according to any one of the previous items, wherein the amount of residual organic solvents, in particular t-butyl methyl ether, is 5.0 wt.-% or less, preferably 2 wt.-% or less, more preferably 1.5 wt.-% or less, particularly 0.5 wt.-% or less, or 0.1 wt.-% or less.
   Forms S contains only small, preferably insignificant or even no detectable amounts of t-butyl methyl ether, and further preferably no detectable amounts of other organic solvents.
5. The crystalline Form S according to any one of the preceding items, which is essentially polymorphically pure, preferably has an X-ray powder diffraction pattern wherein the peaks defined in item 1, optionally further the peaks defined in item 2, represent the peaks with higher intensity compared to other detectable peaks, particular preferred, Form S has no detectable amounts of a crystalline form of venetoclax other than Form S.
6. The crystalline Form S according to any one of the preceding items, which is essentially pure, preferably contains venetoclax in an amount of 99.5 wt.-%, preferably 99.8 wt.-%.
   Chemical purity, including solvent content, is determined by high performance liquid chromatography (HPLC) as described herein.
7. The crystalline Form S according to any one of the preceding items, wherein the peak at the 2-theta angle of 11.4±0.2 degrees 2theta has the highest intensity (100%).
8. The crystalline Form S according to any one of the preceding items, showing no event/significant weight loss in the thermogravimetric analysis (TGA) at temperatures of below 250°C.
9. Method for the preparation of the crystalline Form S of any one of items 1 to 8, comprising the step:
   (B) drying Form HyD of venetoclax, characterized by an X-ray powder diffraction pattern with peaks at 2-theta angles of 5.2±0.2 degrees 2theta, 9.2±0.2 degrees 2theta, 15.0±0.2 degrees 2theta, 21.3±0.2 degrees 2theta, 21.8±0.2 degrees 2theta, when using Cu-Kα radiation, at a temperature and for a time period suitable to obtain crystalline Form S.
10. The method according to item 9, wherein drying of Form HyD takes is performed at a temperature of from 15 to 50°C under vacuum for at least 5 h.
   It is possible that the drying step is carried out stepwise, for instance a first drying step is carried out by applying specific conditions e.g. with regard to drying time and temperature, followed by a second drying step that is carried out at different conditions e.g. with regard to drying time and temperature when compared to the respective conditions applied in the first drying step.
   In one embodiment, the drying is performed at ambient temperature, i.e. in a range from 20 to 25°C, and the vacuum is 10 to 1000mmHg, preferably 10 to 100mmHg.
11. The method according to item 9 or item 10, further comprising the step:
   (A) providing crystalline Form HyD by a method comprising the following steps:
      (a) suspending solid venetoclax in t-butyl-methyl ether (MTBE), thereby obtaining a suspension;
      (b) stirring the suspension, thereby obtaining a slurry; and
      (c) filtering the slurry obtained in step (b), thereby obtaining crystalline Form HyD.
   The solid venetoclax substance useful for suspending step (A) (a) preferably is milled, more preferably is ball milled.
   The ball milling parameters are preferably 30 to 60 minutes at a frequency of 40 Hz.
   Preferably, the ball-milled venetoclax is suspended in MTBE using the ranges of about 5% to about 20% w/v, preferably about 15%w/v.
   Upon filtration of the slurry, the solvate desolvates and transforms into the new hydrated form HyD. Atmospheric moisture replaces part of the MTBE in the structure, thus reducing the solvent content. This intermediate form can then be dried to produce Form S.
   Prior art Form F is made in a similar fashion, however by slurrying amorphous venetoclax in ethyl acetate.
12. The method according to item 11, wherein the stirring of step (b) takes place for about 5 h to about 75h at ambient temperature.
   Preferably, stirring takes place for about 10 hours.
13. Use of crystalline Form S of any of items 1 to 8, for the preparation of a pharmaceutical composition of venetoclax, preferably a pharmaceutical composition comprising an amorphous form of venetoclax.
14. The use according to item 13, wherein the venetoclax component of said pharmaceutical composition has a water content of less than 1 wt.-%, preferably less than 0.1 wt.-%, more preferably, there is no detectable amount of water present, based on the weight of the venetoclax component, and/or the venetoclax component of said pharmaceutical composition has an amount of residual organic solvents, in particular t-butyl methyl ether, of 5.0 wt.-% or less, preferably 2 wt.-% or less, more preferably 1.5 wt.-% or less, particularly 0.5 wt.-% or less, or 0.1 wt.-% or less, based on the weight of the venetoclax component.
   The "venetoclax component" is the amorphous form of venetoclax that is present in the pharmaceutical composition.
   In an alternative embodiment, the venetoclax component of said pharmaceutical composition has a water content of less than 1 wt.-%, preferably less than 0.1 wt.-%, more preferably, there is no detectable amount of water present, based on the weight of the pharmaceutical composition, and/or the venetoclax component of said pharmaceutical composition has an amount of residual organic solvents, in particular t-butyl methyl ether, of 5.0 wt.-% or less, preferably 2 wt.-% or less, more preferably 1.5 wt.-% or less, particularly 0.5 wt.-% or less, or 0.1 wt.-% or less, based on the weight of the pharmaceutical composition.
   It is also possible that in one embodiment, the venetoclax component of said pharmaceutical composition has a water content of less than 1 wt.-%, preferably less than 0.1 wt.-%, more preferably, there is no detectable amount of water present, based on the weight of the pharmaceutical composition and based on the weight of the venetoclax component, and/or wherein the venetoclax component of said pharmaceutical composition has an amount of residual organic solvents, in particular t-butyl methyl ether, of 5.0 wt.-% or less, preferably 2 wt.-% or less, more preferably 1.5 wt.-% or less, particularly 0.5 wt.-% or less, or 0.1 wt.-% or less, based on the weight of the pharmaceutical composition and based on the weight of the venetoclax component.
15. The use according to item 13 or 14, wherein the pharmaceutical composition has a water content of less than 1 wt.-%, preferably less than 0.1 wt.-%, more preferably, there is no detectable amount of water present, based on the weight of the pharmaceutical composition, and/or wherein the pharmaceutical composition has an amount of residual organic solvents, in particular t-butyl methyl ether, of 5.0 wt.-% or less, preferably 2 wt.-% or less, more preferably 1.5 wt.-% or less, particularly 0.5 wt.-% or less, or 0.1 wt.-% or less, based on the weight of the pharmaceutical composition.
16. The use according to items 13, 14, or 15, wherein the pharmaceutical composition is a solid dispersion of venetoclax, preferably an amorphous solid dispersion.
17. Amorphous venetoclax having a water content of less than 1 wt.-%, preferably less than 0.1 wt.-%, more preferably, there is no detectable amount of water present, based on the weight of the amorphous venetoclax, and/or wherein the amorphous venetoclax has an amount of residual organic solvents, in particular t-butyl methyl ether, of 5.0 wt.-% or less, preferably 2 wt.-% or less, more preferably 1.5 wt.-% or less, particularly 0.5 wt.-% or less, or 0.1 wt.-% or less, based on the weight of the amorphous venetoclax.
   The amorphous form of venetoclax is amorphous as determined and correspondingly identified by X-ray powder diffraction (XRPD/XRD) analysis. It may also, optionally instead of or in addition to XRPD, be determined and identified as amorphous when subjected to physical observation, for instance by Polarized Light microscopy (PLM). As known to a person skilled in the art, under certain circumstances, amorphous materials can appear to have birefringence under the polarized light.
18. Amorphous venetoclax obtainable or obtained by conversion of crystalline Form S of venetoclax of any of items 1 to 8 into an amorphous form of venetoclax, wherein said conversion is performed in the absence of solvents and by applying a method that results in amorphous venetoclax, preferably by melting the crystalline Form S.
   Methods that can be applied in order to convert a crystalline form such as crystalline Form S into amorphous venetoclax are known to a person skilled in the art. An example of such a method is melting. Thus, in a preferred embodiment, the conversion of crystalline Form S is carried out by melting the crystalline Form S. The amorphous form of venetoclax can be determined as disclosed elsewhere herein.
19. Crystalline Form HyD of venetoclax, represented by Formula I (4-(4-{[2-(4-chlorophenyl)-4,4-dimethylcyclohex- 1 -en- 1 -yl]methyl}piperazin-1 -yl)-N-({3-nitro-4-[(tetrahydro-2H-pyran-4-ylmethyl)amino]phenyl}sulfonyl)-2-(1H-pyrrolo[2,3-b]pyridin-5-yloxy)benzamide) 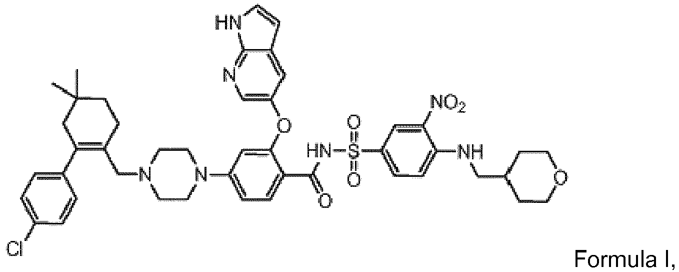 characterized by an X-ray powder diffraction pattern with peaks at 2-theta angles of 5.2±0.2 degrees 2theta, 9.2±0.2 degrees 2theta, 15.0±0.2 degrees 2theta, 21.3±0.2 degrees 2theta, 21.8±0.2 degrees 2theta, when using Cu-Kα radiation.
20. The crystalline Form HyD of the previous item, wherein the X-ray powder diffraction pattern has peaks at 2-theta angles of 5.2±0.2 degrees 2theta, 7.9±0.2 degrees 2theta, 9.2±0.2 degrees 2theta, 10.5±0.2 degrees 2theta, 11.5±0.2 degrees 2theta, 12.3±0.2 degrees 2theta, 13.7±0.2 degrees 2theta,14.6±0.2 degrees 2theta, 15.0±0.2 degrees 2theta, 15.8±0.2 degrees 2theta, 16.2±0.2 degrees 2theta, 16.5±0.2 degrees 2theta, 17.0±0.2 degrees 2theta, 17.4±0.2 degrees 2theta, 17.7±0.2 degrees 2theta, 18.1±0.2 degrees 2theta, 18.6±0.2 degrees 2theta, 19.9±0.2 degrees 2theta, 21.3±0.2 degrees 2theta, 21.8±0.2 degrees 2theta, 22.3±0.2 degrees 2theta, 22.9±0.2 degrees 2theta, 24.0±0.2 degrees 2theta, 24.9±0.2 degrees 2theta, 26.3±0.2 degrees 2theta, and 30.6±0.2 degrees 2theta, when using Cu-Kα radiation.
21. The crystalline Form HyD according to item 19 or 20, which is essentially polymorphically pure, preferably has an X-ray powder diffraction pattern wherein the peaks defined in item 15 represent the peaks with highest intensity, particularly preferred, Form HyD has no detectable amounts of a crystalline form of venetoclax other than Form HyD.
22. The crystalline Form HyD according to any one of items 19 to 21, which is essentially pure, preferably contains venetoclax in an amount of at least 99.8 wt.-%, preferably at least 99.9 wt.-% or more preferably >99.9% wt.-%.
23. The crystalline Form HyD according to any one of items 19 to 22, having an X-ray powder diffraction pattern comprising the peaks as shown in Fig. 6, preferably having an X-ray powder diffraction pattern with peaks and peak intensities as shown in Fig. 7.
   The crystalline Form HyD of the present invention (referred to herein also as "Form HyD") is a hydrated, and solvated crystalline form. It shows desirable properties, e.g., when compared to the known Form F of venetoclax of WO 2012/071336 A1. For instance, it is assumed that Form HyD exhibits improved desolvation behavior, e.g., when compared to known Form F. This can for instance further contribute to improved processability of Form HyD, thereby making Form HyD especially suitable e.g. for further processing such as for conversion into Form S.
24. Method for the preparation of the crystalline Form HyD of any one of items 19 to 23, comprising the following steps:
   (a) suspending solid material of Formula I (venetoclax) in t-butyl-methyl ether (MTBE), thereby obtaining a suspension;
   (b) stirring the suspension, thereby obtaining a slurry;
   (c) filtering the slurry obtained in step (b), thereby obtaining crystalline Form HyD.
   Preferably, the venetoclax of Formula I is a free base solid of venetoclax.
   The solid material of Formula I useful for suspending step (a) preferably is milled, more preferably is ball milled.
   The ball milling parameters are preferably 30 to 60 minutes at a frequency of 40 Hz.
   Preferably, the ball-milled venetoclax is suspended in MTBE using the ranges of about 5% to about 20% w/v, preferably about 15%w/v.
25. The method according to item 24, wherein the stirring of step (b) takes place for about 70h to 80h at ambient temperature.
26. Use of the crystalline Form HyD as defined in any one of items 19 to 23 as an intermediate for the preparation of crystalline Form S of any one of items 1 to 8.
27. Pharmaceutical composition comprising an amorphous form of venetoclax as defined in item 17 or 18, an amorphous solid dispersion of venetoclax, preferably as disclosed herein, the crystalline Form HyD as defined in any one of items 19 to 23, or the crystalline Form S as defined in any one of items 1 to 8, and one or more pharmaceutically acceptable excipients.
28. The pharmaceutical composition according to item 27, wherein the one or more pharmaceutically acceptable excipient is selected from the group consisting of fillers, disintegrants, binders, lubricants, and surfactants; preferably, the pharmaceutical composition comprises the amorphous form of venetoclax as the sole pharmaceutically active ingredient.
29. The pharmaceutical composition according to item 27 or 28, comprising an amorphous solid dispersion of venetoclax, obtainable or obtained by using the crystalline Form S as defined in any one of items 1 to 8 as the starting material in a melt extrusion process in the absence of solvents, i.e. without adding solvents.
   Since the melt extrusion process is performed in the absence of solvents, the amounts of solvents in the amorphous solid dispersion essentially corresponds to the amounts of solvents present in the starting materials used for preparing the amorphous solid dispersion.
   The excipients and venetoclax used for the melt extrusion process are essentially free of solvents, i.e. only contain equilibrium humidity.
   Preferably, the amorphous venetoclax that is contained in the amorphous solid dispersion has a water content of less than 1 wt.-%, preferably less than 0.1 wt.-%, more preferably, there is no detectable amount of water present, based on the weight of the amorphous venetoclax, and/or the amorphous venetoclax has an amount of residual organic solvents, in particular t-butyl methyl ether, of 5.0 wt.-% or less, preferably 2 wt.-% or less, more preferably 1.5 wt.-% or less, particularly 0.5 wt.-% or less, or 0.1 wt.-% or less, based on the weight of the amorphous venetoclax.
   Preferably, the amorphous solid dispersion comprising the amorphous venetoclax that is contained in the pharmaceutical composition, has a water content of less than 1 wt.-%, preferably less than 0.1 wt.-%, more preferably, there is no detectable amount of water present, based on the weight of the amorphous solid dispersion, and/or the amorphous solid dispersion has an amount of residual organic solvents, in particular t-butyl methyl ether, of 5.0 wt.-% or less, preferably 2 wt.-% or less, more preferably 1.5 wt.-% or less, particularly 0.5 wt.-% or less, or 0.1 wt.--% or less, based on the weight of the amorphous solid dispersion.
   Preferably, the pharmaceutical composition comprising the amorphous solid dispersion has a water content of less than 1 wt.-%, preferably less than 0.1 wt.-%, more preferably, there is no detectable amount of water present, based on the weight of the pharmaceutical composition, and/or the pharmaceutical composition has an amount of residual organic solvents, in particular t-butyl methyl ether, of 5.0 wt.-% or less, preferably 2 wt.-% or less, more preferably 1.5 wt.-% or less, particularly 0.5 wt.-% or less, or 0.1 wt.-% or less, based on the weight of the pharmaceutical composition.
   Preferably, the minimum content of venetoclax in the amorphous solid dispersion is 10 wt.-%, 20 wt.-%, 30 wt.-%, or 40 wt.-%. Preferably, the maximum content of venetoclax in the amorphous solid dispersion is 70 wt.-%, 60 wt.-%, 50 wt.-%, or 40 wt.-%. For example, the content of venetoclax in the amorphous solid dispersion is between 10 wt.-% and 70 wt.-%, between 20 wt.-% and 70 wt.-%, or between 30 wt.-% and 70 wt.-%.
30. The pharmaceutical composition according to any one of items 27 to 29, wherein the amorphous solid dispersion is formed with amorphous venetoclax and at least one polymer selected from the group consisting of polyvinyl pyrrolidone (PVP), polyvinyl alcohol (PVA), polyacrylic acid (PAA), poly(ethylene glycol) (PEG), poly(ethylene oxide) (PEO), copovidone, hypromellose acetate succinate (HPMC-AS), polyacrylates, cellulose, cellulose derivatives and mixtures thereof.
   Preferably, no further ingredients are used for preparing said amorphous solid dispersion.
   Preferably, the polymer is a hydrophilic polymer, preferably cellulose or a cellulose derivative. Hydrophilic polymers include, but are not restricted to, cellulose derivatives selected from the group consisting of alkylcellulose, preferably methylcellulose, ethylcellulose, or propylcellulose; hydroxyalkylcellulose, preferably hydroxymethylcellulose, hydroxyethylcellulose, or hydroxypropylcellulose; hydroxyalkylalkylcellulose, preferably hydroxyethylmethylcellulose (HEMC), or hydroxypropylmethylcellulose (HPMC); carboxyalkylcellulose, preferably carboxymethylcellulose (CMC), carboxymethylhydroxyethylcellulose (CMHEC), hydroxyethylcarboxymethylcellulose (HECMC); sodium carboxymethylcellulose, cellulose acetate phthalate (CAP), hydroxypropylmethylcellulose acetate (HPMCA), hydroxypropylmethylcellulose phthalate (HPMCP), hydroxypropylmethylcellulose acetate succinate (HPMCAS), and a mixture of two or more thereof.
31. The pharmaceutical composition according to item 29 or 30, further comprising one or more pharmaceutically acceptable excipients selected from the group consisting of diluents, sweeteners, buffering agents, glidants, flowing agents, flavouring agents, lubricants, preservatives, surfactants, wetting agents, binders, disintegrants, and thickeners.
   In a preferred embodiment, the pharmaceutical composition comprises amorphous venetoclax as the sole pharmaceutically active ingredient.
32. Form S of any one of items 1 to 8, Form HyD of any one of items 19 to 23, amorphous venetoclax of item 17 or 18, or pharmaceutical composition of any one of items 27 to 31, for use in a method of preventing or treating a disease that is characterized by apoptotic dysfunction and/or overexpression of an anti-apoptotic Bcl-2 family protein.
33. Form S of any one of items 1 to 8, Form HyD of any one of items 19 to 23, amorphous venetoclax of item 17 or 18, or pharmaceutical composition of any one of items 27 to 31, for use according to the previous item, wherein the disease is a neoplastic, immune, or autoimmune disease.
34. Form S of any one of items 1 to 8, Form HyD of any one of items 19 to 23, amorphous venetoclax of item 17 or 18, or pharmaceutical composition of any one of items 27 to 31, for use according to items 32 or 33, wherein the neoplastic disease is selected from the group consisting of cancer, mesothelioma, bladder cancer, pancreatic cancer, skin cancer, cancer of the head or neck, cutaneous or intraocular melanoma, ovarian cancer, breast cancer, uterine cancer, carcinoma of the fallopian tubes, carcinoma of the endometrium, carcinoma of the cervix, carcinoma of the vagina, carcinoma of the vulva, bone cancer, colon cancer, rectal cancer, cancer of the anal region, stomach cancer, gastrointestinal (gastric, colorectal and/or duodenal) cancer, chronic lymphocytic leukemia, esophageal cancer, cancer of the small intestine, cancer of the endocrine system, cancer of the thyroid gland, cancer of the parathyroid gland, cancer of the adrenal gland, sarcoma of soft tissue, cancer of the urethra, cancer of the penis, testicular cancer, hepatocellular (hepatic and/or biliary duct) cancer, primary or secondary central nervous system tumor, primary or secondary brain tumor, Hodgkin's disease, chronic or acute leukemia, chronic myeloid leukemia, lymphocytic lymphoma, lymphoblastic leukemia, follicular lymphoma, lymphoid malignancies of T-cell or B-cell origin, melanoma, multiple myeloma, oral cancer, non-small-cell lung cancer, prostate cancer, small-cell lung cancer, cancer of the kidney and/or ureter, renal cell carcinoma, carcinoma of the renal pelvis, neoplasms of the central nervous system, primary central nervous system lymphoma, non-Hodgkin's lymphoma, spinal axis tumors, brain stem glioma, pituitary adenoma, adrenocortical cancer, gall bladder cancer, cancer of the spleen, cholangiocarcinoma, fibrosarcoma, neuroblastoma, retinoblastoma and combinations thereof.
35. Form S of any one of items 1 to 8, Form HyD of any one of items 19 to 23, amorphous venetoclax of item 17 or 18, or pharmaceutical composition of any one of items 27 to 31, for use according to any one of items 32 to 34, wherein the neoplastic disease is a lymphoid malignancy.
36. Form S of any one of items 1 to 8, Form HyD of any one of items 19 to 23, amorphous venetoclax of item 17 or 18, or pharmaceutical composition of any one of items 27 to 31, for use according to any one of items 32 to 35, wherein the lymphoid malignancy is non-Hodgkin's lymphoma, chronic lymphoid leukemia, or acute lymphocytic leukemia.

### Pharmaceutical compositions

The pharmaceutical compositions of the present invention comprising the forms of venetoclax of the present invention may further comprise one or more pharmaceutically acceptable excipients. Such excipients are preferably selected from the group consisting of diluents, sweeteners, buffering agents, glidants, flowing agents, flavouring agents, lubricants, preservatives, surfactants, wetting agents, binders, disintegrants and thickeners. Other excipients known in the field of pharmaceutical compositions may also be used. Furthermore the pharmaceutical composition may comprise a combination of two or more excipients also within one of the members of the above mentioned group.

Suitable wetting agents which can be used for the pharmaceutical compositions of the present invention comprising the forms of venetoclax of the present invention comprise e.g. sodium lauryl sulphate, sodium dioctyl sulfosuccinate, sodium starch glyocolate or wetting agents belonging to the group of the polyethylene glycol sorbitan fatty acid esters, such as wetting agents known as Tween, e.g. Tween 20, 60 and 80.

Suitable binders which can be used for the pharmaceutical compositions of the present invention comprising the forms of venetoclax of the present invention further comprise e.g. alkylcelluloses such as methylcellulose, hydroxyalkylcelluloses such as hydroxymethylcellulose, hydroxyethylcellulose, hydroxypropylcellulose and hydroxybutylcellulose, hydroxyalkylalkylcelluloses such as hydroxyethylmethylcellulose and hydroxypropylmethylcellulose, carboxyalkylcelluoses such as carboxymethylcellulose, alkali metal salts of carboxyalkylcelluloses such as sodium carboxymethylcellulose, carboxyalkylalkylcelluloses such as carboxymethylethylcellulose, carboxyalkylcellulose esters, starches such as starch 1551, pectins such as sodium carboxymethylamylopectin, chitin derivatives such as chitosan, heparin and heparinoids, polysaccharides such as alginic acid, alkali metal and ammonium salts thereof, carrageenans, galactomannans, tragacanth, agar-agar, gum arabic, guar gum and xanthan gum, polyacrylic acids and the salts thereof, polymethacrylic acids and the salts thereof, methacrylate copolymers, polyvinylalcohol, polyvinylpyrrolidone, copolymers of polyvinylpyrrolidone with vinyl acetate, polyalkylene oxides such as polyethylene oxide and polypropylene oxide and copolymers of ethylene oxide and propylene oxide, e.g. poloxamers and poloxamines, copovidone.

Suitable diluents which can be used for the pharmaceutical compositions of the present invention comprising the forms of venetoclax of the present invention further comprise e.g. calcium carbonate, dibasic calcium phosphate, dibasic calcium phosphate dihydrate, tribasic calcium phosphate, calcium sulphate, microcrystalline cellulose including silicified microcrystalline cellulose, powdered cellulose, dextrates, dextrin, dextrose excipient, fructose, kaolin, lactitol, lactose anhydrous, lactose monohydrate, mannitol, sorbitol, starch, modified starch, sodium chloride, sucrose, compressible sugar, confectioner's sugar, a spray-dried mixture of lactose monohydrate and microcrystalline cellulose (75:25), commercially available as Microcelac®, a co-processed spray-dried mixture of microcrystalline cellulose and colloidal silicon dioxide (98:2), commercially available as Prosolv®.

Suitable glidants which can be used for the pharmaceutical compositions of the present invention comprising the forms of venetoclax of the present invention further comprise e.g. talc, colloidal silicon dioxide, starch, and magnesium stearate.

Suitable disintegrants which can be used for the pharmaceutical compositions of the present invention comprising the forms of venetoclax of the present invention further comprise e.g. starch, ion exchange resins, e.g. Amberlite, cross-linked polyvinylpyrrolidone, modified cellulose gum, e.g croscarmellose sodium, sodium starch glycolate, sodium carboxymethylcellulose, sodium dodecyl sulphate, modified corn starch, microcrystalline cellulose, magnesium aluminium silicate, alginic acid, alginate and powdered cellulose.

Suitable lubricants which can also be used for the pharmaceutical compositions of the present invention comprising the forms of venetoclax of the present invention further comprise e.g. magnesium stearate, calcium stearate, stearic acid, talc, polyethylene glycol, sodium lauryl sulphate and magnesium lauryl sulphate.

In addition, the pharmaceutical compositions of the present invention comprising the forms of venetoclax (crystalline forms and amorphous venetoclax) of the present invention may further comprise other optional excipients such as, for example, colouring agents.

### Oral dosage forms

In the following, examples of possible oral dosage forms such as tablets are disclosed:
A preferred tablet of the present invention comprises the forms of venetoclax of the present invention, mannitol, microcrystalline cellulose, sodium starch glycolate, hydroxypropylmethyl cellulose, and magnesium stearate.

Another preferred tablet of the present invention comprises the forms of venetoclax of the present invention, lactose monohydrate, silicified microcrystalline cellulose, croscarmellose sodium, polysorbate 20 (Tween 20), polyvinylpyrrolidone K30 (PVP K30), and magnesium stearate.

A further preferred tablet of the present invention comprises the forms of venetoclax of the present invention, microcrystalline cellulose, polysorbate 20 (Tween 20), polyvinylpyrrolidone K30 (PVP K30), dibasic calcium phosphate (dihydrate or anhydrate e.g. Emcompress^{®} or anhydrous Emcompress^{®}), magnesium stearate, and starch.

In addition a preferred tablet of the present invention comprises the forms of venetoclax of the present invention, microcrystalline cellulose, lactose monohydrate, polysorbate 20 (Tween 20), polyvinylpyrrolidone K30 (PVP K30), magnesium stearate, and starch.

Another preferred tablet of the present invention comprises the forms of venetoclax of the present invention, microcrystalline cellulose, modified starch, polysorbate 20 (Tween 20), polyvinylpyrrolidone K30 (PVP K30), and magnesium stearate.

In one embodiment, suitable tablets may be composed as follows - percentages indicated are w/w-%:

| | |
|---|---|
| 3-8% | the forms of venetoclax of the present invention |
| 35-45% | anhydrous calcium hydrogen phosphate |
| 15-25% | corn starch |
| 2-6% | copovidone |
| 20-30% | microcrystalline cellulose |
| 1-3% | sodium starch glycolate |
| 2-6% | talc |
| 0.5-2% | magnesium stearate |

In another embodiment, suitable tablets may be composed as follows - percentages indicated are w/w-%:

| | |
|---|---|
| approximately 5% | the forms of venetoclax of the present invention |
| approximately 39% | anhydrous calcium hydrogen phosphate |
| approximately 20% | corn starch |
| approximately 3% | copovidone |
| approximately 25% | microcrystalline cellulose |
| approximately 3% | sodium starch glycolate |
| approximately 4% | talc |
| approximately 1% | magnesium stearate |

In the following the present invention will be described in further detail by illustrative, nonlimiting examples.

### Examples

### Comparative Example 1: Preparation of Form F (Example 9 of WO2012/071336)

Venetoclax (1g) was suspended in ethyl acetate (20mL) at room temperature. The suspension was magnetically stirred for 16h and filtered. The damp material was allowed to dry at ambient temperature for 5h and was then analysed by XRD. XRD indicated the predominant phase to be Form F with traces of Form A (incomplete desolvation).

### Comparative Example 2: Drying of Form F

The material from Comparative Example 1 was dried for 7h under vacuum at ambient temperature and then for an additional 17h.

The resulting material was analysed by XRD and found to consist of Form F with traces of Form A. Gas chromatography (GC) indicated an ethyl acetate content of 8.2% after 24h under vacuum.

### Comparative Example 3: Preparation of crystalline Form A (according to WO2012/071336)

901 mg of ball milled venetoclax were dissolved in ethyl acetate (16.5 mL, added in different aliquots), followed by direct precipitation. The slurry was stirred at room temperature for 19 h and filtered. The filter cake was dried under vacuum (30 mbar) at room temperature for 5 h and then at 40 °C for 7 h, yielding crystalline Form A of venetoclax (817 mg) as a yellow powder.

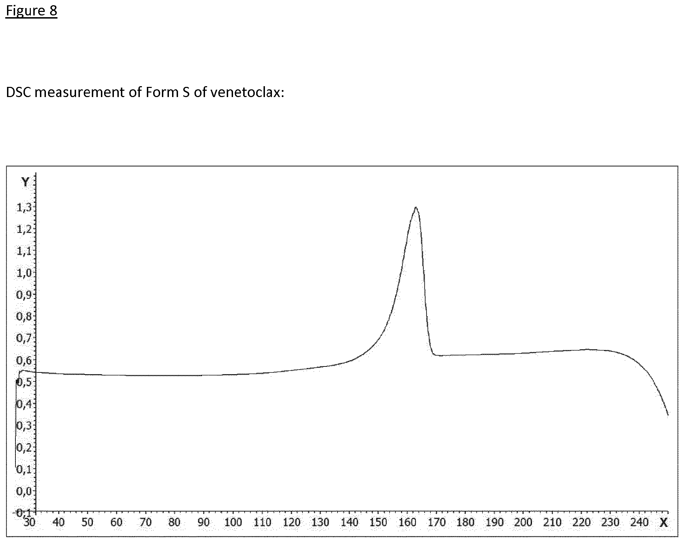

A representative DSC curve (Method 2) of the obtained Form A of venetoclax is shown in Figure 8 and shows a melting endotherm with an onset temperature of about 134°C, a peak maximum at about 140°C and a heat of fusion of about 20 J/g.

### Example 1: Preparation of Form HyD

### Step 1-1:

Two ball mill jars were filled to 30 % of the volume with venetoclax which was a mixture of Form A and the methanol solvate of venetoclax described in WO 2012/071336. To each jar, a milling ball was added and the jars were securely closed. The jars were then tightly screwed into place on the ball mill. The milling was set to take place for 30 minutes at a frequency of 30 Hz. A sample was then taken and analysed by XRPD. This was then repeated to ensure fully amorphous material was produced. A second sample was then analysed by XRPD for amorphous content.

Material produced from the above method (ball milling) was found to be amorphous by XRPD. HPLC purity of the amorphous material was found to be 97.6 %.

### Step 1-2:

The ball milled venetoclax obtained in step 1-1 (1g) was suspended in t-butyl methyl ether (MTBE) (20mL) and magnetically stirred for c.a. 3 days. The slurry was then filtered; the resulting solid was defined as Form HyD.

### Example 2: Preparation of Form S

Form HyD obtained in Step 1-2 of Example 1 was dried at ambient temperature under vacuum for 7h and then for another 17h. The XRD analysis on the resulting solid indicated the presence of Form S. GC indicated the presence of 1.4% MTBE content.

### Example 3: Characterization of Form HyD and Form S

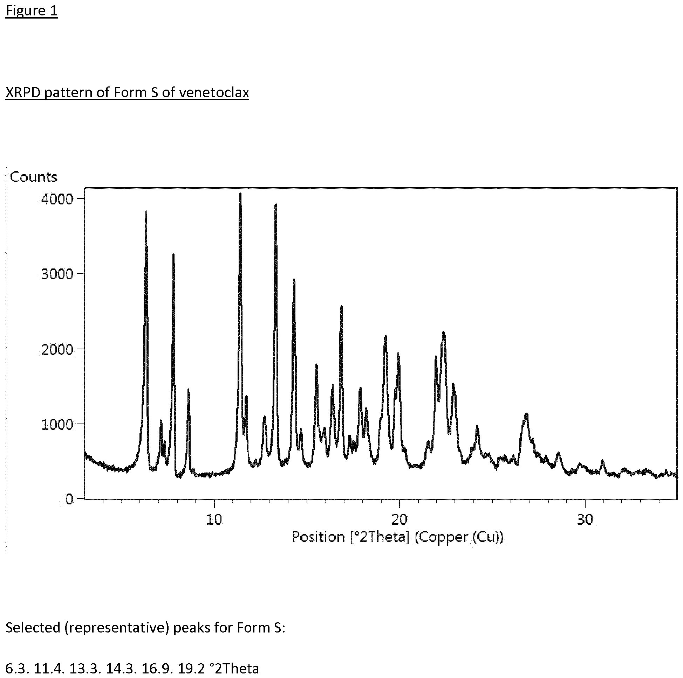

The XRPD pattern of Form S of venetoclax, together with selected (representative) peaks, is shown in Figure 1. The lower part of Fig. 1 lists representative but characteristic peaks of Form S. The upper part of Fig. 1 shows the XRPD pattern of Form S of venetoclax. The corresponding full peak list of Form S of venetoclax is as follows:

**Full peak list of Form S:**

| Pos. [°2Th.] | Rel. Int. [%] | Height [cts] |
|---|---|---|
| 6.3 | 92.2 | 3535.7 |
| 7.1 | 17.7 | 677.9 |
| 7.8 | 75.8 | 2905.7 |
| 8.6 | 29.2 | 1118.5 |
| 11.4 | 100.0 | 3834.9 |
| 11.7 | 27.9 | 1071.3 |
| 12.7 | 20.7 | 792.8 |
| 13.3 | 96.0 | 3682.0 |
| 14.3 | 69.1 | 2649.9 |
| 14.7 | 15.2 | 583.8 |
| 15.5 | 38.5 | 1477.6 |
| 16.4 | 31.2 | 1194.7 |
| 16.9 | 59.3 | 2273.5 |
| 17.9 | 30.8 | 1179.1 |
| 18.2 | 24.0 | 920.1 |
| 19.2 | 49.3 | 1888.7 |
| 19.9 | 43.0 | 1648.0 |
| 22.0 | 41.0 | 1571.5 |
| 22.4 | 47.1 | 1806.6 |
| 22.9 | 31.7 | 1214.5 |
| 24.2 | 16.4 | 627.4 |
| 26.8 | 20.5 | 787.7 |
| 28.6 | 7.3 | 281.0 |
| 29.8 | 3.0 | 116.8 |

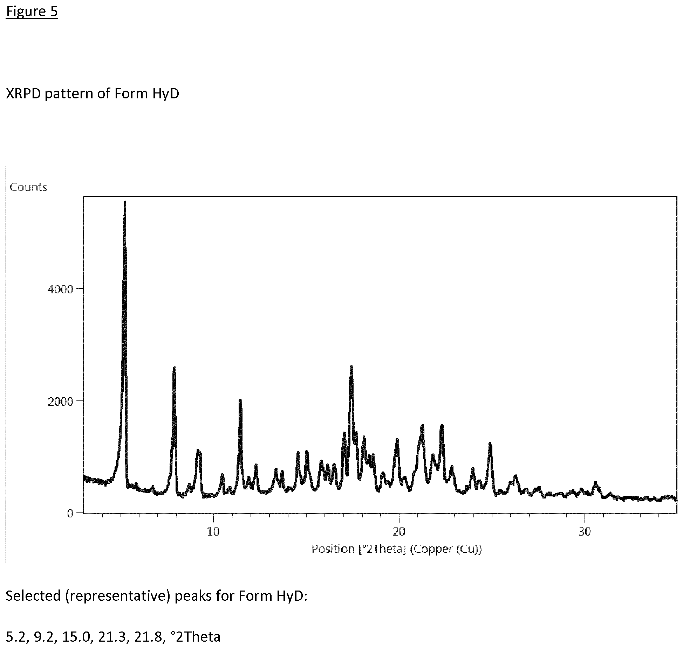

The XRPD pattern of Form HyD of venetoclax, together with selected (representative) peaks, is shown in Figure 5. The lower part of Fig. 5 lists representative but characteristic peaks of Form HyD. The upper part of Fig. 5 shows the XRPD pattern of Form HyD of venetoclax. The corresponding full peak list of Form HyD of venetoclax is shown in Fig. 6.

### Example 4: Preparation of Form S

902 mg of ball milled venetoclax were suspended in 17 mL MTBE. The slurry was stirred at room temperature for 19 h and filtered. The filter cake was dried under vacuum (30 mbar) at room temperature for 5 h and then at 40 °C for 7 h, yielding crystalline Form S of venetoclax (880 mg) as a yellow powder.

A representative DSC curve (Method 2) of the obtained Form S of venetoclax is shown in Figure 9 and shows a melting endotherm with an onset temperature of about 152°C, a peak maximum at about 163°C and a heat of fusion of about 42 J/g.

A comparison of Figures 7 and 8 shows that the melting points and heat of fusions of Form S are both higher than the corresponding values for Form A. This is an indication that Forms A and S are monotropically related and Form S is thermodynamically more stable than Form A at any temperature. This shows that Form S is superior to Form A as regards storage properties.

### Methods

### X-ray powder diffractograms (XRPD/XRD)

The X-ray powder diffractograms (XRPD/XRD) were obtained with an X'Pert PRO diffractometer (PANalytical, Almelo, The Netherlands) equipped with a theta/theta coupled goniometer in transmission geometry, programmable XYZ stage with well plate holder, Cu-Kα1,2 radiation source (wavelength 0.15419 nm) and a solid state PIX'cel detector. The diffractograms were recorded at a tube voltage of 40 kV, tube current of 40 mA. A typical precision of the 2-theta values is in the range of about ± 0.2° 2-theta. Thus a diffraction peak that appears at 5.0° 2-theta can appear between 4.8 and 5.2° 2-theta on most X-ray diffractometers under standard conditions.

The main characteristics of diffraction line profiles are 2θ position, peak height, peak area and shape (characterized by, for example, peak width or asymmetry, analytical function, empirical representation). In addition to the diffraction peaks, an X-ray diffraction experiment also generates a more-or-less uniform background, upon which the peaks are superimposed. Besides specimen preparation, other factors contribute to the background, for instance the sample holder, diffuse scattering from air and equipment, other instrumental parameters such as detector noise, general radiation from the X-ray tube, etc. The peak-to-background ratio can be increased by minimizing background and by choosing prolonged exposure times. In the context of the present invention, the term "peak" denotes a particular 2θ position, wherein the signal-to-noise ratio (calculated according to item 2.2.46 of the European Pharmacopoeia) is greater than 3/1. "Absence of a peak" is herein defined as a peak having an intensity of at most 1%, such as 0.5% or 0.2%, of the highest peak in an XRPD of a sample of venetoclax, more preferably no detectable XRPD peak above background signals.

### Water content - Karl Fischer Coulometric Titration (KF)

The water content can be determined as follows: Approximately 20-25 mg of solid material was accurately weighed into a volumetric flask. The solid was then dissolved in ca. 5 mL of anhydrous methanol and manually introduced into the titration cell of a Mettler Toledo C30 Compact Titrator using a syringe. The syringe was back-weighed after the addition of the solid and the weight of the added sample entered on the instrument. The water content was calculated automatically by the instrument as a percentage and the data printed.

Separately ca. 25 mg of solid material was accurately weighed into a vial. Ca. 2 mL of the Titrator Coulomat was then removed from the system and added to the vial to dissolve the solid. This was then manually introduced into the introduced into the titration cell of a Mettler Toledo C30 Compact Titrator using a syringe. After the addition of the sample, the weight of the added solid was entered on the instrument. The water content was calculated automatically by the instrument as a percentage and the data printed.

### High Performance Liquid Chromatography-Ultraviolet Detection (HPLC-UV)

### Standard Preparation

Accurately weigh 10 mg of standard into a 20 mL volumetric flask and make up to volume with Diluent. This is the working reference standard at ca. 0.5 mg/mL.

### Sample Preparation

Accurately weigh 10 mg of sample material into a 20 mL volumetric flask and make up to volume with diluent. The final concentration is ca.0.5 mg/mL.

### 0.1 % Sensitivity Solution

Prepare an LOQ solution at 0.1% of the nominal standard concentration e.g. accurately pipette 1 mL of standard solution into a 100 mL volumetric flask, make to volume in diluent to give a 1% solution. Further dilute this 1% solution 1 mL to 10 mL to give a 0.1% LOQ solution.

| | |
|---|---|
| Instrument: | Agilent 1100/1200 with UV Detector |
| Column: | Ace Excel 3 C18-PFP, 3 µm 75 x4.6 mm (LC/164) |
| Column Temperature: | 30 °C ± 2 °C |
| Autosampler Temperature: | Ambient |
| UV wavelength: | 275 nm |
| Injection Volume: | 5 µL |
| Flow Rate: | 1.500 mL/min |
| Mobile Phase A: | 0.1 % Trifluoroacetic acid in Deionised Water |
| Mobile Phase B: | 0.1 % Trifluoroacetic acid in Acetonitrile |

Gradient program:

| **Time (minutes)** | **Solvent B [%]** |
|---|---|
| 0.00 | 5.0 |
| 0.50 | 5.0 |
| 10.00 | 100.0 |
| 12.00 | 100.0 |
| 12.10 | 5.0 |
| 15.00 | 5.0 |

### Differential Scanning Calorimetry (DSC)

Method 1: Approximately, 5 mg of material was weighed into an aluminium DSC pan and sealed non-hermetically with a pierced aluminium lid. The sample pan was then loaded into a Seiko DSC6200 (equipped with a cooler) cooled and held at 20°C. Once a stable heat-flow response was obtained, the sample and reference were heated to 240 °C at scan rate of 10°C/min and the resulting heat flow response monitored.

Method 2: Differential scanning calorimetry (DSC) was performed on a Mettler Polymer DSC R instrument. The sample was heated in a 40 microL aluminum pan with pierced aluminum lid from 25 to 250°C at a rate of 10°C/min. Nitrogen (purge rate 50 mL/min) was used as purge gas.

### TGA measurement

The TGA instrument used to test a crystalline form was a Seiko Exstar TG/DTA6200. Approximately 5 mg of material was weighed into an open aluminium pan and loaded into a simultaneous thermogravimetric/differential thermal analyser (TG/DTA) and held at room temperature. The sample was then heated at a rate of 10 K/min from 25 °C to 300 °C during which time the change in sample weight was recorded. Nitrogen was used as the purge gas, at a flow rate of 100 cm³/min.

### Dynamic Vapour Sorption (DVS)

Approximately 20 mg of sample was placed into a mesh vapour sorption balance pan and loaded into a DVS-1 dynamic vapour sorption balance by Surface Measurement Systems. The sample was subject to a ramping profile from 40 - 90 % relative humidity (RH) at 10 % increments, maintaining the sample at each step until a stable weight had been achieved. After completion of the sorption cycle, the sample was dried using the same procedure to 0 % RH and then a second sorption cycle back to 40 % RH was carried out. The weight change during the sorption/desorption cycles were plotted, allowing for the hygroscopic nature of the sample to be determined. XRPD analysis was then carried out on the remaining solid.

### Residual organic solvent

The residual organic solvent can be determined by gas chromatography (GC) using the following method]

| | |
|---|---|
| Column: | DB-624 30 m × 0.32 mm, 1.8 film diameter CG/002 |
| Diluent: | DMSO |
| GC Helium flow: | 30.0 mL/min |
| GC Air flow: | 330.0 mL/min |
| Autosampler Temperature: | Ambient |
| Flow Rate: | 1.500 mL/min |

**Gradient program:**

| **Time (minutes)** | **GC Temperature (°C)** |
|---|---|
| 0.00 | 35.0 |
| 1.000 | 35.0 |
| 5.167 | 60.0 |
| 18.917 | 225.0 |
| 18.918 | 35.0 |

## Claims

1. Crystalline Form S of venetoclax, wherein venetoclax is represented by Formula I (4-(4-{[2-(4-chlorophenyl)-4,4-dimethylcyclohex-1 -en- 1 -yl]methyl}piperazin-1 -yl)-N-({3-nitro-4-[(tetrahydro-2H-pyran-4-ylmethyl)amino]phenyl}sulfonyl)-2-(1H-pyrrolo[2,3-b]pyridin-5-yloxy)benzamide) 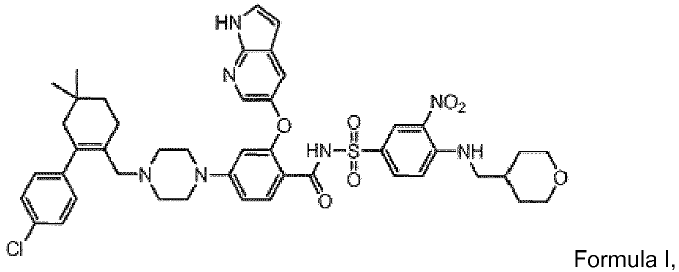 **characterized by** an X-ray powder diffraction pattern with peaks at 2-theta angles of 6.3±0.2 degrees 2theta, 11.4±0.2 degrees 2theta, 13.3±0.2 degrees 2theta, 14.3±0.2 degrees 2theta, 16.9±0.2 degrees 2theta, and 19.2±0.2 degrees 2theta, when using Cu-Kα radiation.

2. The crystalline Form S of claim 1, wherein the X-ray powder diffraction pattern has peaks at 2-theta angles of 6.3±0.2 degrees 2theta, 7.1±0.2 degrees 2theta, 7.8±0.2 degrees 2theta, 8.6±0.2 degrees 2theta, 11.4±0.2 degrees 2theta, 11.7±0.2 degrees 2theta, 12.7±0.2 degrees 2theta, 13.3±0.2 degrees 2theta, 14.3±0.2 degrees 2theta, 14.7±0.2 degrees 2theta, 15.5±0.2 degrees 2theta, 16.4±0.2 degrees 2theta, 16.9±0.2 degrees 2theta, 17.9±0.2 degrees 2theta, 18.2±0.2 degrees 2theta, 19.2±0.2 degrees 2theta, 19.9±0.2 degrees 2theta, 22.0±0.2 degrees 2theta, 22.4±0.2 degrees 2theta, 22.9±0.2 degrees 2theta, 24.2±0.2 degrees 2theta, and 26.8±0.2 degrees 2theta, when using Cu-Kα radiation.

3. The crystalline Form S according to claims 1 or 2, having a water content of less than 1 wt.-%, preferably less than 0.1 wt.-%, and more preferably, there is no detectable amount of water present.

4. The crystalline Form S according to any one of claims 1 to 3, wherein the amount of residual organic solvents is 5.0 wt.-% or less, preferably 2 wt.-% or less, more preferably 1.5 wt.-% or less, particularly 0.5 wt.-% or less.

5. Method for the preparation of the crystalline Form S of any one of claims 1 to 4, comprising the step:
(B) drying Form HyD of venetoclax, **characterized by** an X-ray powder diffraction pattern with peaks at 2-theta angles of 5.2±0.2 degrees 2theta, 9.2±0.2 degrees 2theta, 15.0±0.2 degrees 2theta, 21.3±0.2 degrees 2theta, 21.8±0.2 degrees 2theta, when using Cu-Kα radiation, at a temperature and for a time period suitable to obtain crystalline Form S.

6. The method according to claim 5, further comprising the step:
(A) providing crystalline Form HyD by a method comprising the following steps:
(a) suspending solid venetoclax in t-butyl-methyl ether (MTBE), thereby obtaining a suspension;
(b) stirring the suspension, thereby obtaining a slurry; and
(c) filtering the slurry obtained in step (b), thereby obtaining crystalline Form HyD.

7. Use of crystalline Form S of any of claims 1 to 4, for the preparation of a pharmaceutical composition of venetoclax, preferably a pharmaceutical composition comprising an amorphous form of venetoclax.

8. The use according to claim 7, wherein the venetoclax component of said pharmaceutical composition has a water content of less than 1 wt.-%, preferably less than 0.1 wt.-%, and more preferably, there is no detectable amount of water present, based on the weight of the venetoclax component, and/or wherein the venetoclax component of said pharmaceutical composition has an amount of residual organic solvents, in particular t-butyl methyl ether, of 5.0 wt.-% or less, preferably 2 wt.-% or less, more preferably 1.5 wt.-% or less, particularly 0.5 wt.-% or less, or 0.1 wt.-% or less, based on the weight of the venetoclax component.

9. Crystalline Form HyD of venetoclax, represented by Formula I (4-(4-{[2-(4-chlorophenyl)-4,4-dimethylcyclohex- 1 -en- 1-yl]methyl}piperazin-1-yl)-N-({3-nitro-4-[(tetrahydro-2H-pyran-4-ylmethyl)amino]phenyl}sulfonyl)-2-(1H-pyrrolo[2,3-b]pyridin-5-yloxy)benzamide) 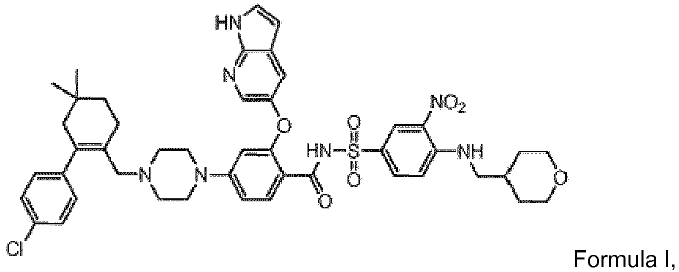 **characterized by** an X-ray powder diffraction pattern with peaks at 2-theta angles of 5.2±0.2 degrees 2theta, 9.2±0.2 degrees 2theta, 15.0±0.2 degrees 2theta, 21.3±0.2 degrees 2theta, 21.8±0.2 degrees 2theta, when using Cu-Kα radiation.

10. The crystalline Form HyD of claim 9, wherein the X-ray powder diffraction pattern has peaks at 2-theta angles of 5.2±0.2 degrees 2theta, 7.9±0.2 degrees 2theta, 9.2±0.2 degrees 2theta, 11.5±0.2 degrees 2theta, 12.3±0.2 degrees 2theta, 14.6±0.2 degrees 2theta, 15.0±0.2 degrees 2theta, 15.8±0.2 degrees 2theta, 16.2±0.2 degrees 2theta, 16.5±0.2 degrees 2theta, 17.0±0.2 degrees 2theta, 17.4±0.2 degrees 2theta, 17.7±0.2 degrees 2theta, 18.1±0.2 degrees 2theta, 18.6±0.2 degrees 2theta, 19.9±0.2 degrees 2theta, 21.3±0.2 degrees 2theta, 21.8±0.2 degrees 2theta, 22.3±0.2 degrees 2theta, and 24.9±0.2 degrees 2theta, when using Cu-Kα radiation.

11. Method for the preparation of the crystalline Form HyD of claim 9 or 10, comprising the following steps:
(a) suspending solid material of Formula I (venetoclax) in t-butyl-methyl ether (MTBE), thereby obtaining a suspension;
(b) stirring the suspension, thereby obtaining a slurry;
(c) filtering the slurry obtained in step (b), thereby obtaining crystalline Form HyD.

12. Use of the crystalline Form HyD as defined in claim 9 or 10 as an intermediate for the preparation of crystalline Form S of any one of claims 1 to 4.

13. Pharmaceutical composition comprising an amorphous solid dispersion of venetoclax, wherein the amorphous venetoclax that is contained in the amorphous solid dispersion has a water content of less than 1 wt.-% based on the weight of the amorphous venetoclax, and/or wherein the amorphous venetoclax has an amount of residual organic solvents of 5.0 wt.-% or less, based on the weight of the amorphous venetoclax; or the crystalline Form HyD as defined in claim 9 or 10; or the crystalline Form S as defined in any one of claims 1 to 4; and one or more pharmaceutically acceptable excipients.

14. The pharmaceutical composition according to claim 13, wherein the amorphous solid dispersion is formed with amorphous venetoclax and at least one polymer selected from the group consisting of polyvinyl pyrrolidone (PVP), polyvinyl alcohol (PVA), polyacrylic acid (PAA), poly(ethylene glycol) (PEG), poly(ethylene oxide) (PEO), copovidone, hypromellose acetate succinate (HPMC-AS), polyacrylates, cellulose, cellulose derivatives and mixtures thereof.

15. Form S of any one of claims 1 to 4, Form HyD of claim 9 or 10, or pharmaceutical composition of claim 13 or 14, for use in a method of preventing or treating a disease that is **characterized by** apoptotic dysfunction and/or overexpression of an anti-apoptotic Bcl-2 family protein.
